# EUROPEAN PATENT APPLICATION

(11) **EP 2 218 403 A1**
(43) Date of publication of application: **18.08.2010**
(21) Application number: 10250257.2
(22) Date of filing: 15.02.2010
(51) Int. Cl.: A61B 17/00, A61B 17/12, A61F 2/84, A61B 17/34

(54) **Delivery catheter handle cover**

(30) Priority: 13.02.2009 US 152527 P
(71) Applicant: Cardiokinetix, Inc., Menlo Park, CA 94025 (US)
(72) Inventor: Khairkhahan, Alexander, Menlo Park California 94025 (US)
(74) Representative: Dee, Ian Mark

(57) **Abstract**

Described herein are handle covers (106) for use with one or more delivery catheters (102) that may be used to deliver and/or remove an expandable cardiac implant. A handle cover may be formed from two interlocking pieces, and may include a control adapter (110) that may be used to control release and/or attachment of an expandable implant. The control adapter may be configured to interact with an implant release control (104) on the delivery catheter. The control adapter may also be lockable to prevent unintended activation of the implant release control. The handle cover may be reusable or disposable (e.g., single-use). In general, the implant control may be shaped so that hit may be held by a doctor, technician or other user, and may facilitate access to various components of the delivery catheter, including one or more ports (e.g., vacuum/inflation ports, etc.).

## Description

### CROSS REFERENCE TO RELATED APPLICATIONS

This application claims priority to U.S. Provisional Patent Application Serial No. 61/152,527, titled "DELIVERY CATHETER HANDLE COVER" and filed on Feb. 13, 2009.

This application may reference any or all of the following US Patent Applications: U.S. Patent Application Serial No. 10/436,959 (titled "SYSTEM FOR IMPROVING CARDIAC FUNCTION") filed May 12, 2003; U.S. Patent Application Serial No. 11/199,633 (titled, "METHOD FOR TREATING MYOCARDIAL RUPTURE") filed Aug. 9, 2005; U.S. Patent Application Serial No. 10/913,608 (titled "VENTRICULAR PARTITIONING DEVICE") filed Aug. 5, 2004; and U.S. Patent Application Serial No. 10/754,182 (titled "VENTRICULAR PARTITIONING DEVICE"), filed Jan. 9, 2004. Each of these patent applications is herein incorporated by reference in its entirety.

### INCORPORATION BY REFERENCE

All publications and patent applications mentioned in this specification are herein incorporated by reference in their entirety to the same extent as if each individual publication or patent application was specifically and individually indicated to be incorporated by reference.

### FIELD OF THE INVENTION

The delivery catheter handles described herein may find use in the area of therapeutic cardiac surgery. In particular, these handle covers may be used with one or more delivery catheters for delivering expandable ventricular implants for apical reconstruction or apical protection.

### BACKGROUND OF THE INVENTION

Implants for partitioning or supporting a portion of a patient's heart, and particularly a ventricle, have been previously described. For example, ventricular partitioning devices are well described in the related applications incorporated by reference above, including US Patent Application Serial No. 10/436,959 (titled "SYSTEM FOR IMPROVING CARDIAC FUNCTION") filed May 12, 2003; US Patent Application Serial No. 11/199,633 (titled, "METHOD FOR TREATING MYOCARDIAL RUPTURE") filed Aug. 9, 2005; US Patent Application Serial No. 10/913,608 (titled "VENTRICULAR PARTITIONING DEVICE") filed Aug. 5, 2004; and US Patent Application Serial No. 10/754,182 (titled "VENTRICULAR PARTITIONING DEVICE"), filed Jan. 9, 2004. These implants may protect or reconstruct the apex of the ventricle, and these implants may therefore be referred to as reconstruction devices or apical protection devices (for use in performing apical reconstruction or apical protection).

Such implants may be delivered and/or positioned using a delivery catheter that is an elongate, typically flexible device adapted to releasably secure to an implant and maintain the implant in the collapsed (delivery) configuration until it is deployed in a desired location within the patient's body. Examples of delivery catheters (which may also be referred to as deploying/deployment systems and delivery systems), are illustrated in these references. The distal end of these delivery catheters is typically adapted to releasably secure to the implant (e.g., partitioning implant), while the proximal end is typically adapted to allow manipulation of the delivery catheter, and particularly to release the device at the distal end. For example, a delivery catheter may include a deployment mechanism and a catheter tube, where the deployment system includes a handle region and a deployment member. The handle may be a molded plastic region that includes an anchor knob and other elements that communicate with the distal end of the deployment catheter.

The handles of such delivery catheters may be difficult to grasp and operate. For example, to save cost, the handle portion may be undersized, and may include numerous ports and controls, including an implant release control. Operation of one or more of these controls may be difficult because of the small size of these controls, and their proximity to other portions of the handle. Thus, it would be beneficial to provide a handle cover, particularly a reusable handle cover that may be fitted over such a delivery catheter to help manipulate the delivery catheter, and therefore enhance delivery of an implant. The systems, methods and devices described herein may address these problems.

Described herein are handle covers, systems including handle covers, and methods of delivering a cardiac implant using a handle cover.

### SUMMARY OF THE INVENTION

In general, the handle covers described herein may be placed over the proximal end of a delivery catheter to aid in manipulation of the delivery catheter and delivery of an expandable implant. Handle covers typically include one or more body regions that secure over a portion of the delivery catheter and one or more control adapters that are configured to engage an implant release control on the delivery catheter. The control adapter may be a button, a switch, a toggle, a knob, a slider, etc. The control adapter may also be lockable to prevent unintentional activation or release of the implant from the delivery catheter. In some variations, the handle cover includes a lock to lock the control adapter. The control adapter may also be configured to provide feedback (e.g., tactile, visible, audible, etc.) to indicate activation of the control adapter. The control adapter may also be configured to regulate the activation of the implant release control of the delivery catheter, for example, by limiting the direction of movement of the implant release control.

A handle cover may be single-use or reusable. For example, a handle cover may be repeatedly secured and released from multiple implant delivery catheters.

The handle covers described herein may be used as part of a system in conjunction with one or more implant delivery catheters. For example, an implant delivery system may include a delivery catheter having and a handle cover. The delivery catheter may include an implant release at a distal portion of the delivery catheter configured to releasably engage an expandable implant, and an implant release control at a proximal portion of the delivery catheter, wherein the implant release control is configured to activate the implant release. The handle cover may include a handle body configured to engage the proximal portion of the delivery catheter, and a control adapter configured to engage the implant release control of the delivery catheter such that activation of the control adapter activates the implant release control.

In general, the handle body may be sued to control the implant delivery. For example, the handle cover (handle body) may include one or more controls for operating an implant release control, implant deployment/expansion control, an implant sheath (e.g., removal/loading), and/or for catheter steering. The controls on the handle cover may interface with controls on the handle. The controls may include sliders, knobs, buttons, etc. In some variations the control of different functions may be combined in a single control (e.g., slider or knob). For example, a control may be a slider with a rotary locking mechanism, or a rotation knob with a sliding lock.

The handle body may include a passageway or opening into which the delivery catheter may fit. A handle body may be formed of two interlocking halves configured to secure over the proximal portion of the delivery catheter; for example, the halves may snap together to secure over the proximal end. The handle body may include an outer surface that is configured to be gripped or otherwise hand-held. The outer surface of the handle body may conform to a users palm or hand, and may be textured to allow gripping. In some variations, the handle body may be shaped to orient the device. For example, the handle cover may include a distinguishable "top," "bottom" and "sides." The handle cover may be configured to secure the implant delivery catheter within the handle body so that it does not rotate significantly relative to the handle body.

The handle cover may comprise any appropriate channels or ports to allow or enhance access to the components of the implant delivery catheter. For example, the handle cover (e.g., the handle body region) may include an inflation port channel through with an inflation port of the implant delivery catheter (e.g., connected to a balloon) may be accessed.

In some variations the control adapter comprises two interlocking halves configured to secure over the implant release control. The control adapter may be coupled to the handle body. The control adapter may be configured to rotate to activate the release of an implant from the delivery catheter. In some variations, the control adapter is configured to be activated by pumping.

In general, any appropriate implant delivery catheter may be used, particularly those including a flexible elongate body and having a distal end including an implant release that is configured to releasably secure to an expandable implant. For example, implant delivery catheters (also referred to herein as "delivery catheters"), and implants that may be released from them are described in US Patent Application Serial No. 10/436,959 (titled "SYSTEM FOR IMPROVING CARDIAC FUNCTION") filed May 12, 2003; US Patent Application Serial No. 11/199,633 (titled, "METHOD FOR TREATING MYOCARDIAL RUPTURE") filed Aug. 9, 2005; US Patent Application Serial No. 10/913,608 (titled "VENTRICULAR PARTITIONING DEVICE") filed Aug. 5, 2004; and US Patent Application Serial No. 10/754,182 (titled "VENTRICULAR PARTITIONING DEVICE"), filed Jan. 9, 2004. In some variations, the implant release comprises a coil that is configured to engage an expandable implant; rotation of this coil may release, e.g., by 'unscrewing' the implant from the catheter. The implant delivery catheter may also include an inflatable balloon at the distal end to help deploy the expandable implant. In some variations, the implant delivery catheter also includes one or more channels for release of a material (e.g., a filling material) through, behind, or into the implant. An implant delivery catheter typically also includes an implant release control at the proximal portion of the implant delivery catheter for controlling the release of an implant from the delivery catheter.

In any of the systems described herein an implant may also be included. Any appropriate implant may be used, particularly those described in the references incorporated above. Expandable (e.g., self-expanding) implants are particularly useful. For example, an implant may have a plurality of expandable ribs or arms that are configured to secure to the wall of a ventricle. In some variations, the implants are partitioning implants that include one or more membranes that may be expanded to partition the heart (e.g., ventricle) of a patient when delivered.

As mentioned, the control adapter may be configured to lock to prevent activation of the implant release control. For example, the handle cover may include a lock mechanism that is connected to the control adapter of the handle cover and prevent it from activating the implant release control of the delivery catheter until it is unlocked. In some variations, the control adapter is configured to be locked and unlocked by pushing or pulling the control adaptor relative to the handle body.

Any of the systems described herein may also include an implant loader that is configured to collapse the expandable implant and engage with the delivery catheter. An implant loader may be a funnel-shaped implant loader. Other implant loaders may be lariat or constrictable loops, hooks for attaching to constricting threads on the implant, or the like. The handle cover may be adapted to secure to the implant loader. For example, a handle cover may include an attachment region on the distal region of the handle cover for connecting to an implant loader. The implant loader may be attached to handle cover in order to keep out of the way, e.g., after it has been used to load an implant onto the delivery catheter, etc.

Also described herein are handle covers for implant delivery catheters. A handle cover may be separate from a system including a delivery catheter. In particular, a handle cover may be adapted for use with more than one delivery catheter. For example, a handle cover may be removable after being secured to a delivery catheter, and may then be secured over a different (or the same) delivery catheter. These handle covers may be substantially the same as those described above. For example, a handle cover may include a handle body configured to secure over a proximal portion of a delivery catheter and a control adapter coupled to the handle body and configured to engage an implant release control of the delivery catheter such that activation of the control adapter causes activation of the implant release control and release of an expandable implant from a distal end of the delivery catheter, wherein the control adapter is configured to lock to prevent activation of the implant release control.

As mentioned above, the handle body may include two interlocking halves configured to secure over the proximal portion of the delivery catheter. The control adapter may comprise two interlocking halves configured to secure over the implant release control. The control adapter may be configured to rotate relative to the handle body. The control adapter may be configured to provide feedback as the control adapter is activated (e.g., tactile, visual, aural, etc. feedback).

In some variations, the handle cover further comprises an attachment site at the distal end of the handle body for attaching an implant loader, wherein the implant loader is configured to collapse the implant. The handle cover may also include a catheter exit port at the proximal portion of the handle body. In some variations, the handle cover includes multiple exit ports. The control adapter may be configured to rotate in one direction to activate the implant release control. In some variations, the control adapter is configured to unlock when pulled proximally.

Also described herein are methods of delivering an implant to target region of a patient's heart. For example, the method may include the steps of: advancing an expandable implant that is coupled to the distal portion of an implant delivery catheter in a collapsed configuration to a target region of the patient's heart; manipulating a control adapter portion of a handle cover to activate an implant release control on the implant delivery catheter, wherein the handle cover is coupled to the proximal portion of the delivery catheter; and releasing the expandable implant into the target region of the patient's heart, whereby a plurality of ribs on the expandable implant expand to secure the implant within the target region of the patient's heart.

In some variations, the method may also include the step of securing two interlocking halves of the handle cover over the proximal end of the implant delivery catheter.

The method may also include the step of unlocking the control adapter of a handle cover.

In general, the target region comprises a ventricle or a portion within a ventricle (e.g., the apex region, etc.).

The method may also include the step of securing the handle cover over the proximal portion of the delivery catheter so that the control adapter engages the implant release control of the delivery catheter.

The step of manipulating the control adapter may comprise rotating the control adapter, or otherwise moving the control adapter to activate the implant release control on the implant delivery catheter. This step may also include providing feedback indicating the activation of the implant release control.

The step of releasing the expandable implant may include rotating a coil at the distal end of the delivery catheter until the coil disengages from the implant. This step may also include partitioning the left ventricle of the subject's heart into productive and non-productive regions.

Also described herein are implant delivery catheters including a distal tip guard. The distal tip guard may be referred to as a screw guard in variations of the delivery catheter having a screw or other threaded region at the distal end. The distal tip guard is typically an extension from the distal end of the device (e.g., beyond the screw or threaded region) that is made of a soft material, and can prevent or reduce damage to the heart wall from the delivery catheter.

### BRIEF DESCRIPTION OF THE DRAWINGS

FIG. 1 shows one embodiment of an implant delivery system for improving cardiac function including a handle cover.

FIG. 2 shows an embodiment of delivery catheter handle cover as described herein.

FIG. 3 is a perspective view of one example of a base portion of the handle body of a handle cover.

FIG. 4 is a perspective view of one variation of a control adaptor of a handle cover.

FIG. 5 is a partial cut-away perspective view of an annular snap feature of a control adaptor and a handle body of a handle cover.

FIG. 6 shows a perspective view of a handle cover illustrating one variation of an implant loader detent on the handle body of a handle cover.

FIG. 7 illustrates one embodiment of an implant delivery system for improving cardiac function including a handle cover as described herein.

FIG. 8A shows the distal end of an implant delivery catheter including a screw (distal tip) guard. FIG 8B illustrates an enlarged view of the distal end of the implant delivery catheter shown in FIG. 8A.

### DETAILED DESCRIPTION OF THE INVENTION

A handle cover for delivery catheter typically includes a handle body region configured to cover a portion of the delivery catheter such as the proximal handle portion of the delivery catheter, and a control adapter that is configured to engage an implant release portion of the delivery catheter. A handle cover may be reusable or single-use, and may be constructed so that it can encase or sheath the proximal end of the delivery catheter while enhancing the function of the delivery catheter. For example, the handle cover may be formed of two or more interlocking parts that secure over the delivery catheter handle.

For example, in one embodiment, a handle cover for an implant delivery catheter includes a handle cover body configured to cover and engage with a proximal portion of a delivery catheter. The handle cover also includes a control adaptor. A control adapter may be a knob, switch, slider, dial, button, etc. that can be manipulated by a user holding the handle to release an implant into a target region of a patient's heart. The control adaptor typically activates an implant release control that is located on the proximal portion of the implant delivery catheter, enabling the release of the implant from the distal portion of the deliver catheter.

A handle cover may be part of an implant deliver system that includes a delivery catheter, handle cover, and (optionally) an implant configured to be releasably secured at a distal portion of the delivery catheter. The delivery catheter typically includes an implant release control located at the proximal end of the delivery catheter that can be activated to release (or in some variations, engage) an implant by the distal end region of the delivery catheter. When the handle cover is secured to the delivery catheter, the implant release control may be activated by the control adaptor. The control adapter of the handle cover may also regulate the operation of the implant release control. For example, the control adapter may include a limiter that limits the rate and/or amount that the implant release control is activated. In some variations, the control adapter is lockable, to prevent release of an implant from the delivery catheter by activation of the implant release control until it is unlocked. In some variations, the handle cover includes an indicator of the activation state of the implant release control. An indicator may be visible (e.g., by a marking, text, color change, etc.) showing the amount of activation or release of an implant by the delivery catheter.

In operation, a handle cover may be used with a delivery catheter to control the deployment of an implant from the delivery catheter. For example, a distal portion of an implant delivery catheter may be advanced near a target region of the patient's heart, such as with the patient's ventricle. An expandable implant may be attached to the distal end of the implant delivery catheter in a collapsed configuration. In variations of the handle cover including a lockable control adapter, the control adapter of the handle cover may then be unlocked. The control adapter can then be manipulated (e.g., by rotation) to activate the implant release control. The activation of the implant release control causes release of the expandable implant. Accordingly, the expandable implant may be released into the target region of the patient's heart. Releasing the expandable implant may involve partitioning the left ventricle of the subject's heart into productive and non-productive regions.

In some variations, the activation of the implant release control includes releasing an expandable implant by rotating a threaded region or coil at the distal end of the delivery catheter until the coil disengages from an implant. For example, an implant may have a threaded receiving region that engages the release mechanism on the distal end of the delivery catheter.

An appropriate implant may be used with the delivery catheter and/or handle covers described herein. For example, refer to the implants described in the patent applications incorporated by reference above. In particular, expandable implants having a plurality of ribs to help secure the implant within the target region of the patient's heart (e.g., ventricle) are of particular interest. Partition implants, which may include one or more membranes for partitioning a region of the heart, are also of interest.

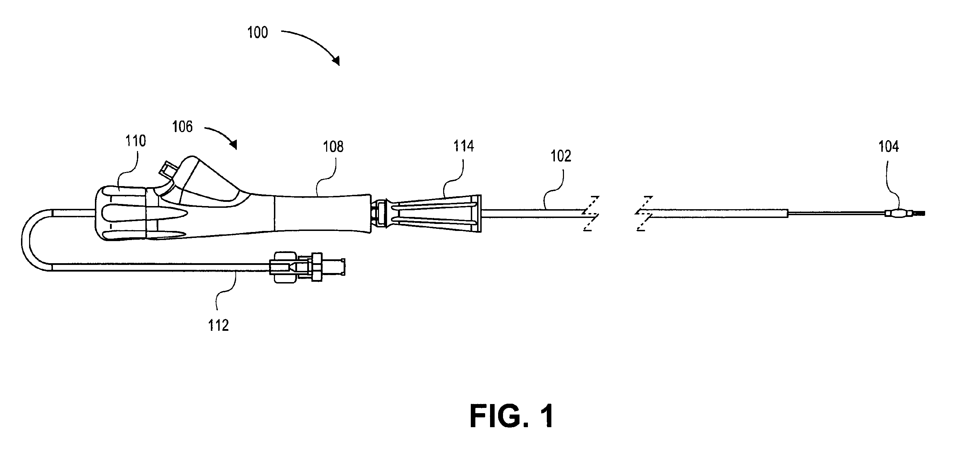

FIGS. 1-7 illustrate variations of handle cover and systems including handle covers. For example, FIG. 1 illustrates an implant delivery system for improving cardiac function. The implant delivery system 100 includes a delivery catheter 102, an implant release 104, a handle cover 106, an implant loader 114 and inflation port 112.

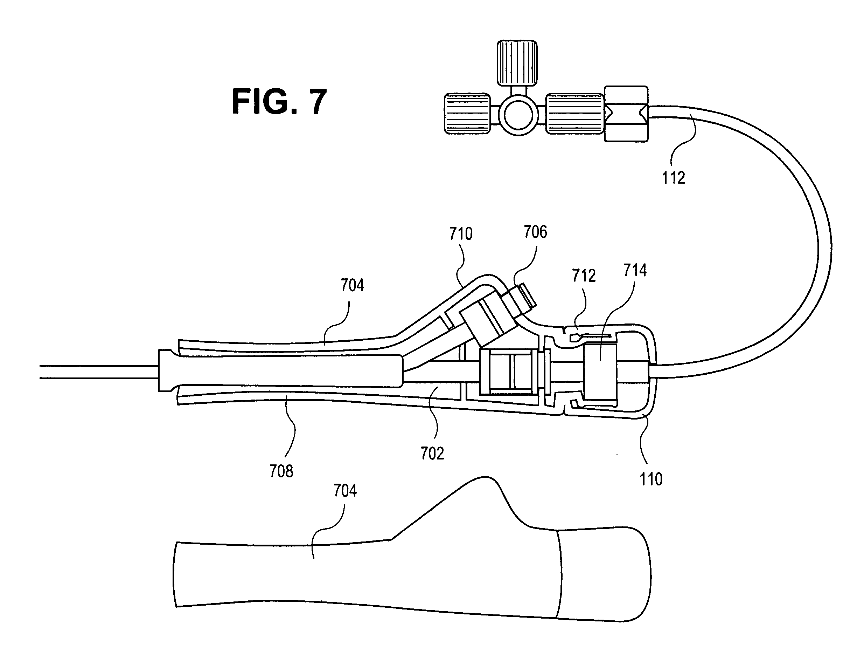

The implant delivery system 100 shown in FIG. 1 includes an implant release 104 at a distal portion of the delivery catheter. The implant release 104 may be activated by an implant release control, as describe in greater detail below (e.g., see FIG. 7). The implant release 104 may be connected to the implant release control of the delivery catheter (not visible in FIG. 1) by a wire (e.g., guidewire) or another mechanism. The implant release 104 mechanism typically releases an implant. In addition, the delivery catheter may also be used to deliver material such as coils and/or a bioabsorbable materials within or behind the implant after it is positioned. For example, the delivery catheter may include a channel or passage for delivery of material through, behind or into the implant. For example, the delivery catheter may allow delivery of a bioabsorbable material such as collagen, gelatin, polylactic acid, polyglycolic acid, copolymers of polylactic acid and polyglycolic acid, polycaprolactone, mixtures and copolymers, or the like. In some variations this material may be delivered behind the device after it has been implanted.

In some variations, the implant release control (not visible in FIG. 1) rotates the implant release mechanism 104, enabling the implant release 104 to release an implant such as those depicted in US Patent Application Publication No. 2006/0281965 and US Patent Application Publication No. 2006/0264980, which are herein incorporated by reference in their entirety.

An implant delivery system 100 may also include an implant loader 114. An implant loader 114 can collapse an implant. The implant loader may also engage with the delivery catheter 102 particularly when the implant loader is not in use (e.g., before or after collapsing the implant and loading to the delivery catheter). The implant loader 114 may have a funnel or cone shape, as illustrated in the variation shown in FIG. 1.

As mentioned above, any appropriate delivery catheter for delivering a collapsible implant may be used. For example, the delivery catheter 102 may take the form described in US Patent Application Publication No. 2006/0281965 or US Patent Application Publication No. 2006/0264980.

As describe in FIG. 7, a delivery catheter 102 may include two tubular members, attaching to an inflation port 112 and a coil delivery port 706. As shown in Fig. 7, the inflation port 112 and the coil delivery port 706 are located at a proximal portion of the delivery catheter. The inflation port 112 may be configured to inflate a balloon to help secure the implant within the target region of the patient's heart. The coil delivery port 706 may be used to release the implant and to rotate a coil at the distal end of the delivery catheter until the coil disengages from the implant. Optionally, the coil delivery port may be replaced by another type of delivery port.

As mentioned above, and illustrated in FIG. 1, a handle cover 106 may include a handle body 108 and a control adaptor 110. The handle body 108 in this example is formed of two interlocking pieces. Alternatively, the handle body 108 may be one complete piece or more than two interlocking pieces. The handle body 108 may be made of plastic, glass or other materials.

As depicted in FIG. 7, the handle body 108 in this example forms an opening or passageway 702 generally configured to hold the proximal portion of the delivery catheter 102. This opening or passageway 702 is formed of an elongate section 708, a projecting section 710 and a base 712. The elongate section 708 of the passageway 702 encases the proximal portion of the delivery catheter 102. The projecting section 710 is configured to hold the portion of the delivery catheter 102 that is attached to a coil delivery port. The projecting section 710 also serves as a gripping mechanism for the implant delivery system 100.

The handle body 108 may also include gripping tabs, detents, attachment and projections to secure and/or engage portions of the delivery catheter 102 throughout the elongate section 708, the projecting section 710 and the base 712. Thus, the delivery catheter may be secured within the handle cover so that it doesn't rotate or slide with respect to the handle cover.

As illustrated in FIGS. 1-3, a control adaptor 110 may be attached to the handle body 108 and is configured to engage the implant release control of the delivery catheter 102. In this example, the control adaptor 110 forms a knob that can be rotated to control the deployment. Optionally, the control adaptor 110 may be a pumping, electrical, or other control mechanism to engage the implant release control of the delivery catheter 102.

A user may operate the control adaptor 110 to activate the release of an implant from the delivery catheter. Activating the control adapter typically activates the implant release control (714 in FIG. 7).

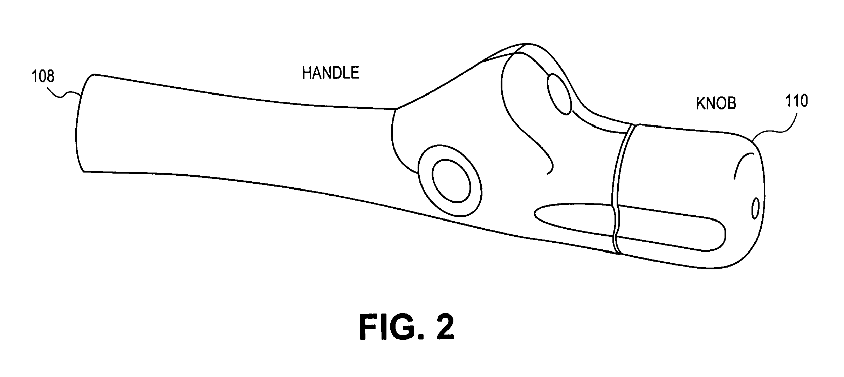

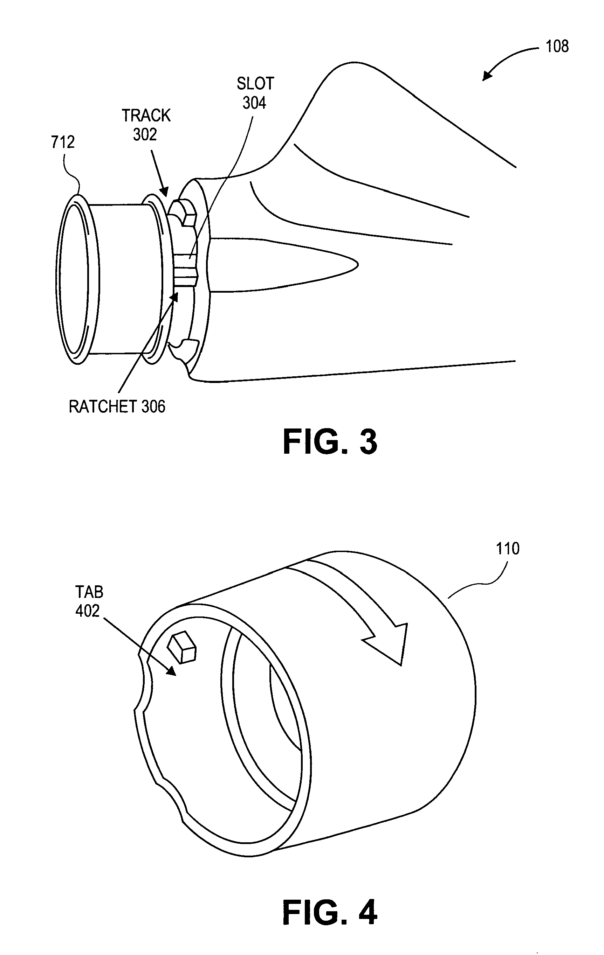

FIG. 2 shows a side perspective view of a handle cover including a control adapter 110 at the proximal end. FIG. 3 shows a partial view of the handle body region of the handle cover shown in FIG. 2. The base 712 of the handle body 108 is shown separated from the control adaptor 110. The base 712 includes a track 302, a slot 304 and a ratchet 306. FIG. 4 illustrates a control adaptor 110 including a tab 402.

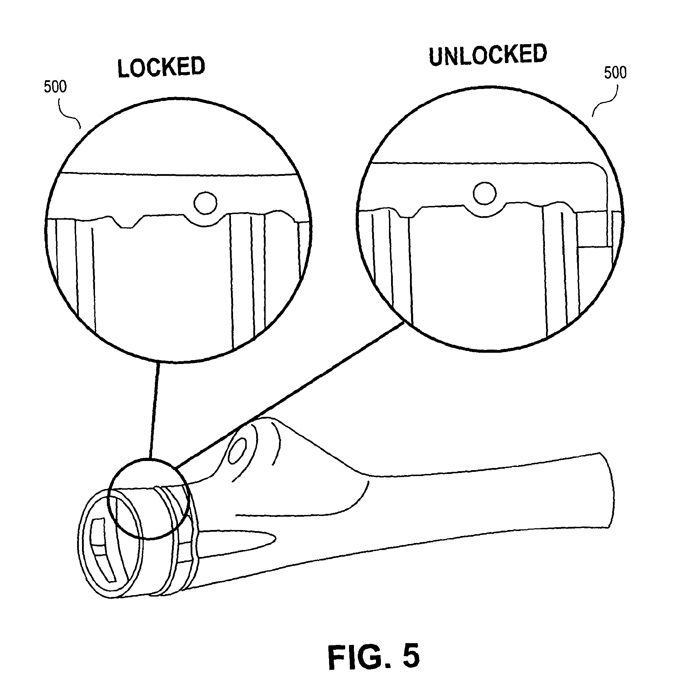

The control adaptor 110 shown in FIG. 4 is configured to rotate in a counter clockwise direction (and also in part in a clockwise direction). The control adaptor 110 is also configured to be locked and unlocked. Activation of the control adapter 110 causes the implant release control to activate the implant release, thereby enabling an implant to release from a distal end of the delivery catheter. The control adaptor 110 can be locked to prevent activation of the implant release control when manipulating the control adaptor 110. When the control adapter 110 is locked, e.g., when the tab 402 on the control adaptor 110 is locked into the slot 304 on the handle body 108 in the example shown in FIGS. 2-4, the control adapter may be rotated but will not engage the implant release control and release the implant. This locking mechanism may prevent the control adaptor 110 from rotating, or may simply decouple rotation of the control adapter from the implant release control. As shown in FIG. 5, the control adaptor 110 may be held in the locked position by the annular snap feature 500 on the control adaptor 110 and the handle body 108.

In FIG. 5, the control adaptor 110 can be unlocked by a distal pull; in some configurations the control adapter is unlocked by pushing distally, or by some combination of pushing/pulling and rotating. When the control adaptor 110 is put into the unlocked position, the annular snap feature 500 on the control adaptor 110 and the handle body 108 may hold the control adaptor 110 in the unlocked position. Accordingly, the tab 402 can slide counterclockwise through the track 402 on the handle body 108. As the control adaptor 110 is moved from the locked to the unlocked position, the ratchet 306 on the handle body 108 interferes with tab 402 so that the control adaptor 110 cannot be rotated in the clockwise direction.

One side of the slot 304 is formed by the ratchet 306 on the handle body 108. The ratchet 306 on the handle body 108 allows the tab 402 on the handle body 108 to pass in the counterclockwise direction, but not in the clockwise direction. After each full turn in the counterclockwise direction, the tab 402 snaps past the ratchet 306 and the control adaptor 110 is unable to make any subsequent turns in the clockwise direction.

The control adaptor 110 may also provide feedback upon rotation or activation of the implant release control 104. Feedback may indicate that the control adapter is engaged (or locked), and may indicate the status of the control adapter/implant release control. For example, in some variations, the deployment of the implant may involve the removal of a threaded attachment member/deployment member. A feedback on the handle may indicate how far into the deployment (e.g., how unscrewed) the implant is. For example the handle cover may indicate by audible 'clicks' how many turns of the deployment mechanism have been completed. The feedback may be visual, aural, tactile, or the like. For example, the control adaptor 110 may provide clicking sounds to the operator of the implant delivery system 100.

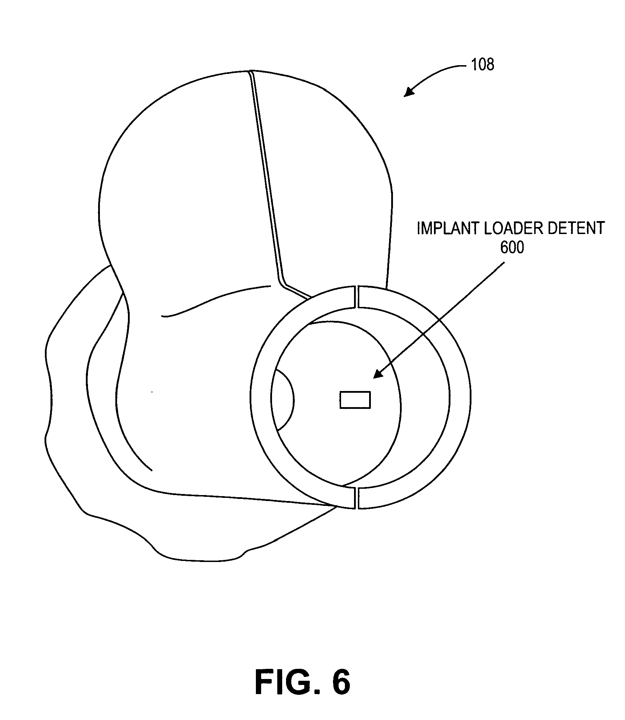

FIG. 6 illustrates an implant loader detent 600 on a distal end of the handle body 108. Each interlocking piece of the handle body 108 includes the implant loader detent 600 to secure the implant loader 114. When the implant delivery system 100 is not in use, the implant loader 114 is in the distal section of the handle body 108.

### Distal Tip Guard

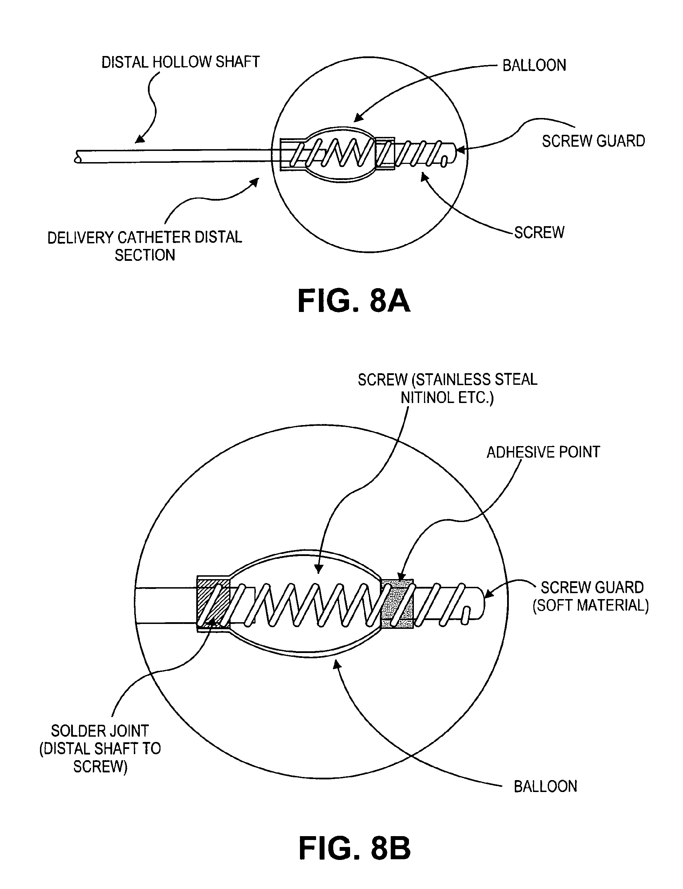

FIG. 8A and 8B show the distal end of an implant delivery catheter including a distal tip guard. In this variation, the distal tip guard may also be referred to as a screw guard, because the distal end of the delivery catheter has an implant release that includes a threaded region (shown as a coil or screw) projecting from the distal end, to which the implant may be releasably engaged. This screw region may be secured at or near the distal end of the implant delivery catheter along with an inflatable (balloon) region that also forms a portion of the implant release. Distal to the implant release is a screw guard, as illustrated in FIGS. 8A and 8B.

In general, the distal tip guard is a cap, protrusion or projection from the distal end of the device that prevents the implant release portion at the distal end of the implant delivery catheter from damaging the heart wall. In FIGS. 8A and 8B the distal tip guard is a screw guard that projects distally from the screw region of the implant release portion, and is a soft material (e.g., a material having a low durometer, such as low durometer Pebax, etc.). The screw guard may be connected to the distal end of the device in any appropriate manner, including adhesively, (e.g., via an adhesive joint), by crimping, sealing, welding, or the like. The distal end of the screw guard may be blunted or otherwise atraumatic.

To the extent not otherwise described herein, the various components of the implants, applicators/delivery catheters, and handle covers may be formed of conventional materials and in a conventional manner as will be appreciated by those skilled in the art.

While particular forms of the invention have been illustrated and described herein, it will be apparent that various modifications and improvements can be made to the invention. Moreover, individual features of embodiments of the invention may be shown in some drawings and not in others, but those skilled in the art will recognize that individual features of one embodiment of the invention can be combined with any or all the features of another embodiment. Accordingly, it is not intended that the invention be limited to the specific embodiments illustrated. It is intended that this invention to be defined by the scope of the appended claims as broadly as the prior art will permit.

## Claims

1. An implant delivery system comprising:
a delivery catheter, the delivery catheter including
an implant release at a distal portion of the delivery catheter configured to releasably engage an expandable implant,
an implant release control at a proximal portion of the delivery catheter, wherein the implant release control is configured to activate the implant release; and
a handle cover including
a handle body configured to engage the proximal portion of the delivery catheter, and
a control adapter configured to engage the implant release control of the delivery catheter such that activation of the control adapter activates the implant release control.

2. The implant delivery system of claim 1, wherein the implant release comprises a coil configured to engage an expandable implant.

3. The implant delivery system of claim 1, wherein the implant release comprises a suture or wire configured to engage an expandable implant.

4. The implant delivery system of any one of claims 1 to 3, wherein the control adapter is configured to lock to prevent activation of the implant release control.

5. The implant delivery system of any one of the preceding claims, further comprising an expandable implant configured to partition a cardiac ventricle.

6. The implant delivery system of any one of the preceding claims further comprising an implant loader, wherein the implant loader is configured to collapse the expandable implant and engage with the delivery catheter.

7. The implant delivery system of any one of claims 1 to 5, further comprising a funnel shaped implant loader, wherein the implant loader is configured to collapse the expandable implant and engage with the delivery catheter.

8. A handle cover for an implant delivery catheter comprising:
a handle body configured to secure over a proximal portion of a delivery catheter; and
a control adapter coupled to the handle body and configured to engage an implant release control of the delivery catheter such that activation of the control adapter causes activation of the implant release control and release of an expandable implant from a distal end of the delivery catheter,
wherein the control adapter is configured to lock to prevent activation of the implant release control.

9. The implant delivery system of any one of claims 1 to 7 or the handle cover of claim 8, wherein the handle body comprises two interlocking halves configured to secure over the proximal portion of the delivery catheter.

10. The implant delivery system of any one of claims 1 to 7 and 9 or the handle cover of claim 8 or 9, wherein the control adapter comprises two interlocking halves configured to secure over the implant release control.

11. The implant delivery system of any one of claims 1 to 7, 9 and 10 or the handle cover of any one of claims 8 to 10, wherein the handle body comprises an inflation port channel.

12. The implant delivery system of any one of claims 1 to 7 and 9 to 11 or the handle cover of any one of claims 8 to 11, wherein the control adapter is configured to rotate or slide.

13. The implant delivery system of any one of claims 1 to 7 and 9 to 12 or the handle cover of any one of claims 8 to 12, wherein the control adapter is configured to be locked and unlocked by pushing, pulling, or rotating the control adaptor relative to the handle body.

14. The implant delivery system of any one of claims 1 to 7 and 9 to 13 or the handle cover of any one of claims 8 to 13, further comprising an implant loader attachment region on the distal region of the handle cover, wherein the implant loader is configured to collapse the implant.

15. The implant delivery system of any one of claims 1 to 7 and 9 to 14 or the handle cover of any one of claims 8 to 14, wherein the control adapter is configured to be activated by pumping.

16. The implant delivery system of any one of claims 1 to 7 and 9 to 15 or the handle cover of any one of claims 8 to 15, wherein the control adapter is configured to provide feedback as the control adapter is activated.

17. The implant delivery system of any one of claims 1 to 7 and 9 to 16 or the handle cover of any one of claims 8 to 16 further comprising a catheter exit port at the proximal portion of the handle body.

18. The implant delivery system of any one of claims 1 to 7 and 9 to 17 or the handle cover of any one of claims 8 to 17 further comprising multiple exit ports.
